# EUROPÄISCHE PATENTSCHRIFT

(11) **EP 3 280 951 B1**
(45) Veröffentlichungstag und Bekanntmachung des Hinweises auf die Patenterteilung: **12.06.2019**
(21) Anmeldenummer: 16724467.2
(22) Anmeldetag: 07.04.2016
(51) Int. Cl.: H01J 61/34, H01J 61/50

(54) **SCHUTZROHR FÜR EINE UV-RÖHRE, INSBESONDERE EINE UVC-RÖHRE**
PROTECTIVE PIPE FOR A UV TUBE, IN PARTICULAR A UV-C TUBE
TUBE DE PROTECTION POUR UN TUBE UV, NOTAMMENT UN TUBE UVC

(30) Priorität: 09.04.2015 DE 102015105362
(43) Veröffentlichungstag der Anmeldung: 14.02.2018
(73) Patentinhaber: Sterilair AG, 8570 Weinfelden (CH)
(72) Erfinder: GRAUPNER, Martin, 8594 Güttingen (CH); SCHLEGEL, Simon, 9220 Bischofszell (CH)
(74) Vertreter: Heisel, Wolfgang
(86) Internationale Anmeldenummer: PCT/IB2016/051967
(87) Internationale Veröffentlichungsnummer: WO 2016/162816

(56) Entgegenhaltungen:
- WO-A1-00/01986
- DE-U1- 20 306 737
- JP-A- 2002 237 209
- US-A- 4 048 537
- US-A1- 2006 113 485

## Beschreibung

### Technisches Gebiet

Die Erfindung bezieht sich auf ein Schutzrohr für eine UV-Lampe für die Aufnahme einer Röhre mit einem stabförmigen Hüllrohr, wobei auf der Umfangsfläche ein Sichtfenster vorgesehen ist, durch das die von der Röhre emittierte Strahlung, insbesondere UVC-Strahlung, austreten kann. Ferner bezieht sie sich auf die Verwendung von Polytetrafluorethylen für eine UV-Lampe.

### Definitionen

UV bedeutet in Bezug auf diese Anmeldung ultraviolett und wird zur Kennzeichnung eines definierten Wellenlängenbereichs verwendet.

Ultraviolette Strahlen bilden einen Teil des natürlichen Sonnenlicht-Spektrums. Kurzwellige ultraviolette Strahlen gehören in die Gruppe der optischen Strahlen. Dennoch sind sie für das menschliche Auge unsichtbar. Die UV-Strahlen aus dem sogenannten C-Band (daher die Bezeichnung UVC) haben um 260 nm (Nanometer) einen stark keimtötenden Effekt. Die Abtötungsrate unterliegt dem Dosis-Wirkungs-Prinzip; deshalb sind technische Kenndaten zur Röhrenleistungen und die Beschreibungsdauer für funktionssichere UVC-Entkeimung auschlaggebend.

Die Keimtötung wirkt insbesondere auf Bakterien, Viren, Hefen sowie Schimmel. Durch eine sogenannte UV-Desinfektion von Luft, Wasser oder Oberflächen werden diese Mikroorganismen wirkungsvoll inaktiviert. Die Technologie der UV-Entkeimung nutzt keine Chemie und keine toxischen Verbindungen und führt nicht zu mutationsbedingten Resistenzbildungen. Die unerwünschten Mikroorganismen werden in Sekundenschnelle inaktiv, während die Produkteigenschaften des bestrahlten Produktes nahezu unverändert bleiben. Die UV-Entkeimung wird insbesondere in den Bereichen eingesetzt, in denen hohe Anforderungen an den Verbraucherschutz und Anforderung durch das HACCP-Konzept bestehen.

Die ultraviolette Strahlung ist sehr energiereich und löst bei Absorption der Strahlung fotochemische Reaktionen in den jeweiligen Nukleinsäuren der exponierten Zelle aus. Liegen zwei Thymine im DNA-Strang nebeneinander, reagieren sie und bilden ein für die Duplikation und Transkription unbrauchbares Dimer. Bei mehreren Schäden ist die Zelle nicht mehr in der Lage sich zu regenerieren. Sie verliert die Fähigkeit zur Teilung und Vermehrung und stirbt.

Unter ökonomischen Gesichtspunkten ist die Keimreduktion von bis zur 5-log Stufe möglich. Die genaue UV-Dosis, die zur Inaktivierung der Mikroorganismen führt, ist dabei typenspezifisch verschieden. Während die überwiegenden Teile Bakterien und Viren mit relativ geringer Dosis inaktiv werden, benötigen Hefen, Schimmel, Pilze und Sporen eine entsprechende höhere Dosis.

Die kurzwellige UVC-Strahlung ist abschirmbar durch gewöhnliches Fensterglas, transparenten Kunststoff, wie beispielsweise Makrolon® oder Acrylglas sowie alle praktisch undurchsichtigen Materialien.

### Stand der Technik

Besonders in der lebensmittelverarbeitenden Industrie wird die UVC-Entkeimung eingesetzt. Aufgrund der Umgebung, in der die Strahlenquellen eingesetzt werden, sind besondere Anforderungen an die UC-Quelle gesetzt. Sie muss mindestens dem IP52 Standard genügen.

Die UVC Quelle ist in der Regel von einem Hüllrohr umgeben. Dieses Hüllrohr besteht aus einem Quarzglas, das die Strahlungsquelle umgibt. Das Hüllrohr mit der UV-Quelle und den entsprechenden Anschlüssen bildet die Röhre. Man spricht, sofern eine UVC Quelle vorgesehen ist, auch von einer UVC Röhre. Die elektrischen Anschlüsse, d.h. die Verbindung von der UVC Quelle zu einem Stromanschluss wird durch einen Sockel gebildet, der entsprechende elektrische Kontakt, vorzugsweise Pins, aufweist. Diese werden steckerartig in eine Aufnahme gesteckt, um einen elektrischen Kontakt herzustellen.

Um das Hüllrohr vor einem Bruch zu schützen und zu vermeiden, dass bei einem Bruch Splitter verstreut werden, ist vorgesehen, dass ein Schutzrohr das Hüllrohr umgibt. Dieses Schutzrohr besteht aus einem vorzugsweise rotationssymmetrischen Grundkörper aus Edelstahl oder Aluminium und ist derart bemessen, dass dieser Grundkörper in einem Abstand das Hüllrohr umgibt. Zudem ist auf der Umfangsfläche ein Sichtfenster vorgesehen, aus dem die von der Strahlungsquelle emittierte Strahlung austritt. Dieses Sichtfenster wird in der Regel gefräst oder gelasert.

Die Innenwandung des Schutzrohrs weist einen Reflektor auf, derart dass die emittierte Strahlung zumindest teilweise konzentriert durch das Sichtfenster tritt. Dieser Reflektor besteht in der Regel aus einer Aluminiumschicht oder einer Aluminiumlegierung, wie beispielsweise Aluminium-Mangan.

Aus der DE 699 17 253 T2 ist eine hocheffiziente Beleuchtungsquelle und ein Beleuchtungssystem mit verbesserter abgegebener Bestrahlungsstärke bekannt. Bei den beschriebenen Ausführungen wird als Strahlquelle eine langestreckte UV-Lichtquelle vorgeschlagen, die von einer extrem stark reflektierenden Umhüllung umgeben ist. Diese Umhüllung ist ein Kunststoffrohr, das eine Aperturöffnung aufweist. Die Grösse dieser Aperturöffnung ist definiert. Dabei ist die Umhüllung derart gestaltet, dass diese hinsichtlich ihrer Wandung dünn ist, damit die nicht reflektierte Strahlung, die in Wärme umgewandelt wird, unmittelbar aus der Umhüllung austreten kann, da diese die Leistung der UV-Quelle negativ beeinflusst.

### Nachteile des Standes der Technik

Durch die Reflexion der UV Strahlung innerhalb des Schutzrohrs geht ein Teil der Leistung der UVC Röhre verloren. Dies bedeutet, dass wenig Leistung durch das Sichtfenster transportiert wird. Bei beispielsweise einer Leistung der Röhre von 58W wird eine emittierte UVC Bestrahlungsstärke von ca. 14mW/cm2 in einem Abstand von 2cm relativ zum Öffnungsfenster erreicht.

Aluminium bzw. Aluminiumlegierung hat einen Reflexionsgrad von ca. 87-89 %. Dadurch ist die emittierte Leistung gering und es ergeben sich dadurch entsprechende Verluste.

Aluminium lässt sich aber als Reflektor nur facettenartig anordnen, eine im Querschnitt runde homogene Fläche entsteht nicht. Dadurch erhält man unterschiedliche Reflexionsflächen, die zu unterschiedlichen Strahlrichtungen führen.

### Aufgabe der Erfindung

Die Aufgabe der Erfindung besteht darin, die emittierte Leistung der UVC-Röhre zu erhöhen, ohne die Röhre oder die UV-Quelle zu ändern und ohne auf die Vorteile des Schutzrohrs zu verzichten.

### Lösung der Aufgabe

Der erste Teil der Grundidee der Lösung besteht darin, ein im Vergleich zum Aluminium oder der Aluminium Legierung besser reflektierendes Material zu verwenden, um damit die emittierte Leistung aus dem Sichtfenster zu erhöhen. Die Lösung besteht darin, Polytetrafluorethylen (Kurzzeichen PTFE, gelegentlich auch Polytetrafluorethen) als Reflektormaterial einzusetzen. Umgangssprachlich wird dieser Kunststoff oft mit dem Handelsnamen Teflon® gekennzeichnet.

Dadurch wird eine hohe Leistungskonstanz der UV-Quelle erreicht.

Ein umgebender durchsichtiger Schlauch schützt auch das Hüllrohr, das durch das Sichtfenster an sich frei zugänglich wäre. Durch die Wahl des Materials des Schutzschlauchs wird die Transmissionsfähigkeit nur geringfügig beeinflusst.

### Vorteile der Erfindung

Der Grundgedanke der Erfindung ist es, ein Material als Reflexionsmaterial für den Reflektor zu verwenden, das über eine Wellenlängen-Bandbreite von 1000nm bis 250nm nahezu gleichbleibend hohe Reflexionseigenschaften aufweist. Diese Eigenschaften weist Polytetrafluorethylen auf.

Eine bevorzugte Lösung besteht darin, Polytetrafluorethylen in optisch reinweißer Qualität als diffus reflektierende Beschichtung zu verwenden. Umgangssprachlich wird dieses auch optisches PTFE genannt.

Das Polytetrafluorethylen besteht aus einer Schicht bis zu 2mm und weist Reflexionseigenschaften von über 97% auf und zwar über die Bandbreite von 1000nm bis zu 250nm. Es ist vorzugsweise weiß und wärmebeständig bis ca. 250 - 260° C; es ist unbrennbar.

Ein weiterer wesentlicher Vorteil in der Verwendung von Polytetrafluorethylen als Reflexionsmaterial ist darin zu sehen, dass das Material wärmeisolierend wirkt. Dies bedeutet, dass die von der UV-Lampe, insbesondere UVC-Quelle erzeugte Wärme in dem Schutzrohr verbleibt. Dadurch entsteht für die UV-Lampe eine geschlossene Umgebung, die nahezu von der Aussentemperatur unabhängig ist. Dies ermöglicht es, die UV-Lampe auch in Bereichen einzusetzen, in denen sie bisher nicht stabil gewesen ist und temperatursensitiv reagiert hat. Die Auswirkung besteht unter anderem darin, dass die emittierte Leistung weit unter der Norm ist. Auf diese Weise kann nun die UV-Quelle auch in kalter oder wechselnder Temperartur-Umgebung eingesetzt werden, so dass sich ein breites Anwendungsspektrum bei hoher Leistungseffizienz ergibt.

Durch die Anordnung des Reflexionsmaterials und damit die Ausbildung als Reflektor wird erreicht, dass mit einem einfach herzustellenden Aluminiumkörper, der Anschlussmöglichkeiten umfasst, ein Reflektor zusätzlich eingeführt werden kann, der zumindest einen Teil der emittierenden Strahlen der UV-Quelle in Richtung eines im Schutzrohr vorgesehenen Sichtfensters lenkt. Das Sichtfenster erstreckt sich in Längsrichtung über einen Teil der Mantelfläche des Schutzrohrs. Dieser Reflektor kann dadurch einen hohen Reflexionsgrad aufweisen, da dieser rotatorisch um das Hüllrohr angeordnet ist (das Material kann so geformt werden und muss nicht facettenartig ausgebildet sein) und aus einem Material besteht, das einen hohen Reflexionsgrad besitzt.

Ein weiterer Vorteil bei der Verwendung von Polytetrafluorethylen liegt darin, dass dieses sehr eng an die UV-Lampe angebracht werden kann, da dieses sehr genau die zylindrische Form des Hüllrohrs abbildet. Der Spalt zwischen dem Hüllrohr und dem Reflektor ist dadurch gering. Dadurch erhöht sich die Reflexionsleistung zusätzlich. Zudem entsteht ein zusätzlicher Wärmeeffekt, der für die UV-Quelle in Bezug auf die emittierte Leistung förderlich ist.

Als eine weiter vorteilhafte Ausbildung ist vorgesehen, dass zumindest der Bereich des Sichtfensters durch ein durchsichtiges Material abgedeckt ist. Da dieses Sichtfenster oder auch jede andere Aperturöffnung eine Möglichkeit bietet, dass trotzdem Einflüsse aus der Umgebung, wie Temperatur, Schmutz etc. in das Schutzrohr eindringen, aber auch die an eine im Schutzrohr vorteilhafte konstante Temperatur nicht gehalten werden kann, ist vorgesehen, vorzugsweise ist über das gesamte Hüllrohr einen durchsichtigen Polytetrafluorethylen Schlauch anzuordnen, dessen Teil sich über das Sichtfenster spannt. Da Polytetrafluorethylen einen sehr geringen Brechungsindex aufweist, ist die Ablenkung oder auch Reflexion der UV-Strahlung, die aus dem Sichtfenster austreten und durch den Schlauch hindurch emittiert, sehr gering. Der Schlauch hüllt somit das gesamte Schutzrohr ein und endet an den Seiten jeweils im Gewindebereich. Da dieser Schlauch auch reissfest ist, kann ein ungewolltes "Einbrechen" in das Sichtfenster nicht erfolgen.

Sollte dennoch einmal das Hüllrohr oder die UV-Quelle selbst defekt sein und zerspringen, so gelangen keine Splitter ausserhalb des Schutzrohrs, da der Schlauch das Schutzrohr einschliesslich des Bereichs des Sichtfensters einschliesst. Damit ist auch ein Einsatz in hochsensiblen Bereichen, wie beispielsweise der Lebensmittelproduktion möglich.

Der Schlauch über dem Schutzohr kann einfach tütenartig überzogen werden und durch Wärmeeinwirkung angeschrumpft werden, so dass ein enges Anliegen an das Schutzrohr erreicht wird, ohne dass beispielsweise Flüssigkeit oder Feuchtigkeit zwischen Innenseite des Schlauchs und Aussenseite des Schutzrohrs gelangen kann.

Alternativ kann auch ein durchsichtiger Kunststoffschlauch mit entsprechender Reissfestigkeit aufgezogen und geschrumpft werden.

Durch die Ausbildung eines Schlauchs kann auch vor das Sichtfenster eine Linse eingesetzt werden, so dass die emittierenden Strahlen fokussiert oder diffus aus dem Sichtfenster heraus ausgegeben werden können.

Überraschend ist festgestellt worden, dass gerade durch die geschlossene Ummantelung der UV-Quelle die Strahlungsleistung unabhängig von der Umgebung gut ist und gerade der gegenteilige Effekt genutzt wird, nämlich die von der Strahlquelle erzeugte Wärme innerhalb des Schutzrohrs zu halten. Da als Reflektor ein Teflonmaterial verwendet wird, das einen hohen Reflexionsgrad hat, wird eine Überhitzung der UV-Quelle dennoch vermieden. Somit ist das Zusammenspiel zwischen Schutzrohr, Reflektor und auch Schlauch entscheidend für den Betrieb einer stabilen UV-Quelle.

Weitere vorteilhafte Ausgestaltungen sind aus der nachfolgenden Beschreibung, den Ansprüchen sowie den Zeichnungen zu entnehmen.

### Zeichnungen

Es zeigt:
- Fig. 1: eine exemplarische perspektivische Ansicht auf die UV-Lampe mit Schutzrohr in Explosionsdarstellung;
- Fig. 2: eine perspektivische Ansicht auf das erfindungsgemässe Schutzrohr
- Fig. 3: eine perspektivische Ansicht auf das Schutzrohr sowie ein das Schutzrohr umgebenden Schlauch, teilweise im Schnitt.

### Beschreibung eines Ausführungsbeispiels

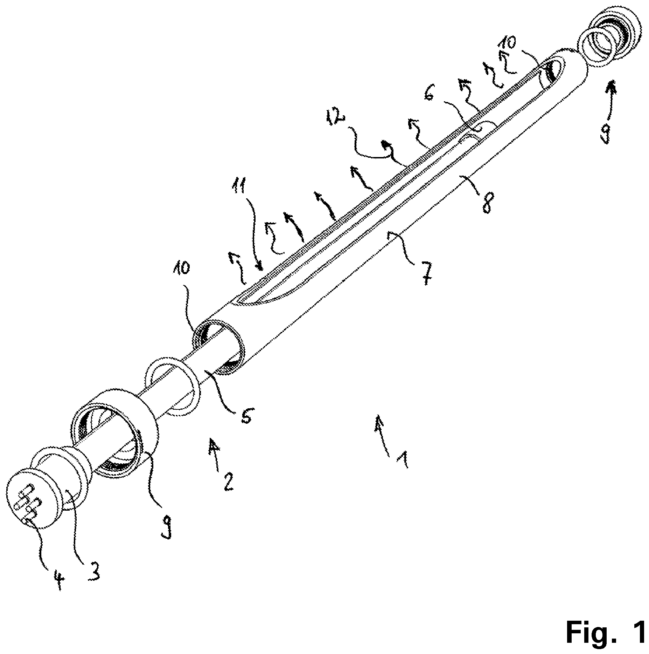

In Fig. 1 ist eine UV-Lampe 1 gezeigt. Diese UV-Lampe 1 besteht aus einer UV-Röhre 2, die wiederum einen Sockel 3 mit einem elektrischen Anschluss 4 umfasst. Mit dem Sockel 3 ist eine UV-Quelle gekoppelt, die in der Zeichnung nicht näher dargestellt ist. Die UV-Quelle ist von einem stabförmigen Hüllrohr 5 umgeben. Das Hüllrohr 5 besteht in der Regel aus einem Quarzglas. Das Ende der UV-Röhre 2, das dem Sockel 3 gegenüberliegt, weist ein Abschlusselement 6 auf.

Ein eine Umfangsfläche 7 aufweisendes Schutzrohr 8 umgibt das Hüllrohr 5. Das Schutzrohr 8 ist rotationssymmetrisch und ist an seinen beiden Stirnseiten geöffnet. Dadurch ist es möglich, das Schutzrohr 8 über das Hüllrohr 5 zu schieben. Bei einem bevorzugten Ausführungsbeispiel, das in der Fig. 1 dargestellt ist, weist das Schutzrohr 8 Elemente 9 zu beiden Seiten auf, die jeweils mit Innengewinden 10 innerhalb des Schutzrohrs 8 zusammenwirken, so dass die UV-Röhre 2 fest innerhalb des Schutzrohrs 8 positionierbar ist. Alternative Befestigungsmittel für die Elemente 9 sind ebenfalls denkbar, wie beispielsweise Magnetverschluss, Bajonettverschluss etc.

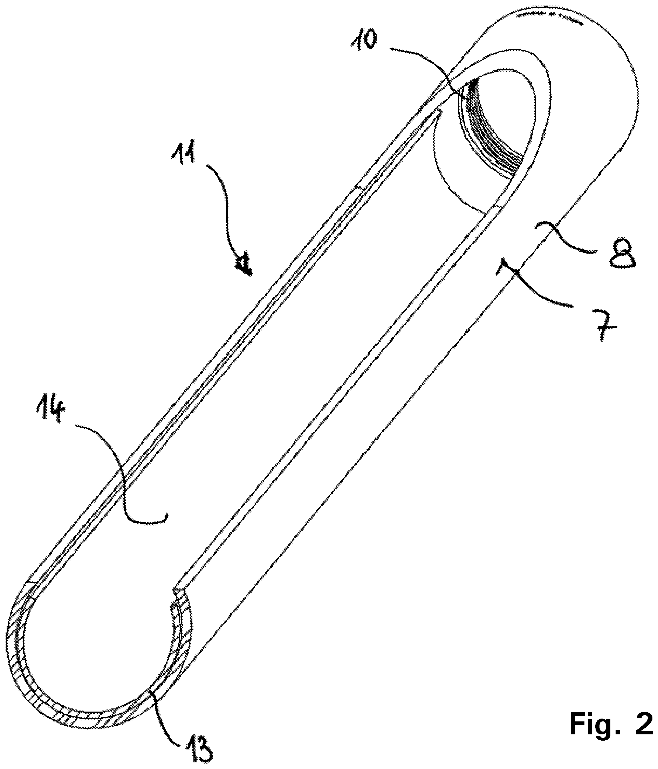

Das Schutzrohr 5 - wie auch in Fig. 2 dargestellt - weist ferner ein Sichtfenster 11 auf. Dieses Sichtfenster 11 erstreckt sich über einen Teilbereich der Umfangsfläche 7 des Schutzrohrs 8. Dieses Sichtfenster 11 gibt den Zugang zu dem Hüllrohr 5 frei und weist keine durchsichtige Bedeckung auf. Dies bringt den Vorteil, dass die Strahlung 12 der UV-Quelle, die aus dem Hüllrohr 4 emittiert, in die dargestellte Richtung freigegeben wird.

Innerhalb des Schutzrohrs 8 ist die Beschichtung 13 von Polytetrafluorethylen angebracht und bildet einen Reflektor 14. Ein Grossteil (ca. 95%) der aus dem Schutzrohr 5 emittierenden Strahlung wird über den Reflektor in die Umgebung reflektiert. Aufgrund der sehr einfachen Verarbeitung von Polytetrafluorethylen kann der Reflektor 14 dem Hüllrohr 5 passend geformt werden. Zudem ist eine sehr nahe Anlage an das Hüllrohr 5 möglich, da eine facettenartige Ausgestaltung, wie sie bei der Verwendung von Aluminium bekannt ist, wegfällt.

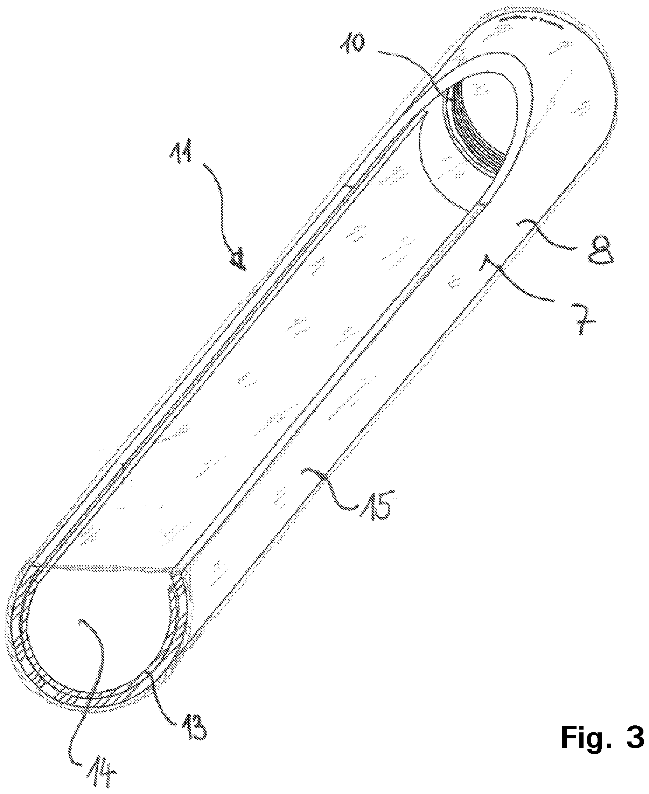

In Fig. 3 ist eine perspektivische Ansicht auf das Schutzrohr 8 gemäss Fig. 2 dargestellt, jedoch mit dem Unterschied, dass dieses mit einem Schutzschlauch 15 umgeben ist. Dieser Schutzschlauch 15, wie er in Fig .3 dargestellt ist, umgibt das Schutzrohr 15 vollständig und liegt eng an dessen Umfangsfläche 7 an. Dieser Schutzschlauch 15 erstreckt sich über die freien Enden des Schutzrohrs15 und wird von den Elementen 9 zusammen mit den entsprechenden Dichtungen aufgenommen. Die Dichtungen liegen eng an der Aussenwandung des Schutzschlauchs 15 an. Damit ist das innenliegende Hüllrohr 5 vollständig abgedichtet. Ist die UV Quelle extremen Umweltbedingungen ausgesetzt, so kann auch Schmutz von aussen nicht an das Hüllrohr gelangen, da das Sichtfenster 11 vollständig mit dem aus transparentem Material bestehenden Schlauch 15 abgedeckt ist. Das Material ist derart gewählt, dass die emittierende Strahlung 12 nicht oder nur geringfügig beeinflusst wird. Vorzugsweise wird ein durchsichtiges Telfonmaterial vorgeschlagen, da dieses auch extrem reissfest ist.

Somit ist ein Schutzrohr 11 mit einem Reflektor 14 sowie einen das Schutzrohr 11 umgebenden Schlauch 15 bereitgestellt, das nahezu unabhängig von dem Einsatzgebiet bezogen auf die Umgebung eine gleichbleibende UV-Stahlqualität und - leistung bereitstellt, da überraschenderweise festgestellt worden ist, dass eine konstante lokale Umgebung für die Strahlquelle einen erheblichen Einfluss auf die emittierende Strahlleistung hat. Zudem kann eine solche UV-Quelle auch in kritischen Bereichen, beispielsweise der Lebensmittelindustrie eingesetzt werden.

### BEZUGSZEICHENLISTE

### Schutzrohr für eine UV-Röhre, insbesondere eine UVC-Röhre

- 1: UV-Lampe
- 2: Röhre
- 3: Sockel
- 4: Anschluss
- 5: Hüllrohr
- 6: Abschlusselement
- 7: Umfangsfläche
- 8: Schutzrohr
- 9: Elemente
- 10: Innengewinde
- 11: Sichtfenster
- 12: Strahlung
- 13: Beschichtung
- 14: Reflektor
- 15: Schlauch

## Patentansprüche

1. Schutzrohr für eine UV-Lampe (1) für die Aufnahme einer Röhre (2) mit einem stabförmigen Hüllrohr (5), wobei auf der Umfangsfläche (7) ein Sichtfenster (11) vorgesehen ist, durch das die von der Röhre (2) emittierte Strahlung (12), insbesondere UVC-Strahlung, austreten kann, wobei auf der Innenseite des Schutzohrs (8) als Reflektor (14) eine Beschichtung (13) aus Polytetrafluorethylen aufgebracht ist, **dadurch gekennzeichnet, dass** das Schutzrohr (8) aus Metall besteht, sowie das Sichtfenster (11) von einem zumindest im Bereich des Sichtfensters (11) zumindest teiltransparentem aus Kunststoff bestehenden Schlauch (15) umgeben ist.

2. Schutzrohr nach Anspruch 1, **dadurch gekennzeichnet, dass** das Schutzrohr (8) aus Edelstahl oder Aluminium besteht.

3. Schutzrohr nach Anspruch 1, **dadurch gekennzeichnet, dass** der Schlauch (15) aus Kunststoff besteht.

4. Schutzrohr nach Anspruch 3, **dadurch gekennzeichnet, dass** der Schlauch (15) aus Polytetrafluorethylen besteht.

5. Schutzrohr nach Anspruch 1 oder 2, **dadurch gekennzeichnet, dass** die UV-Lampe (1) eine UVC-Quelle umfasst.

## Claims

1. Protective tube for a UV lamp (1) for accommodating a fluorescent tube (2) with a rod-shaped casing tube (5), wherein, on the circumferential surface (7), there is provided a window (11) through which the radiation (12), in particular UVC radiation, emitted by the fluorescent tube (2) can emerge, wherein a coating (13) composed of polytetrafluoroethylene is applied as a reflector (14) to the inner side of the protective tube (8), **characterized in that** the protective tube (8) is composed of metal, and the window (11) is surrounded by a hose (15) which, at least in the region of the window (11), is at least partially transparent and is composed of plastic.

2. Protective tube according to Claim 1, **characterized in that** the protective tube (8) is composed of high-grade steel or aluminium.

3. Protective tube according to Claim 1, **characterized in that** the hose (15) is composed of plastic.

4. Protective tube according to Claim 3, **characterized in that** the hose (15) is composed of polytetrafluoroethylene.

5. Protective tube according to Claim 1 or 2, **characterized in that** the UV lamp (1) comprises a UVC source.

## Revendications

1. Tube de protection pour une lampe UV (1) pour recevoir un tube (2) avec un tube d'enveloppe (5) en forme de barre, une fenêtre de vision (11) étant prévue sur la surface périphérique (7), à travers laquelle peut sortir le rayonnement (12) émis par le tube (2), en particulier le rayonnement UVC, un revêtement (13) en polytétrafluoroéthylène étant appliqué sur le côté intérieur du tube de protection (8) en tant que réflecteur (14), **caractérisé en ce que** le tube de protection (8) est en métal et la fenêtre de vision (11) est entourée par un tuyau (15) au moins partiellement transparent, constitué de plastique, au moins dans la région de la fenêtre de vision (11).

2. Tube de protection selon la revendication 1, **caractérisé en ce que** le tube de protection (8) se compose d'acier inoxydable ou d'aluminium.

3. Tube de protection selon la revendication 1, **caractérisé en ce que** le tuyau (15) se compose de plastique.

4. Tube de protection selon la revendication 3, **caractérisé en ce que** le tuyau (15) se compose de polytétrafluoroéthylène.

5. Tube de protection selon la revendication 1 ou 2, **caractérisé en ce que** la lampe UV (1) comprend une source d'UVC.
